(19) Europäisches Patentamt
European Patent Office
Office européen des brevets

(11) Veröffentlichungsnummer: **0 181 442**
**B1**

(12) # EUROPÄISCHE PATENTSCHRIFT

(45) Veröffentlichungstag der Patentschrift: **12.07.89**

(51) Int. Cl.⁴: **C 07 J 5/00**, C 07 J 7/00, C 07 J 1/00, C 07 J 63/00

(21) Anmeldenummer: **85110247.5**

(22) Anmeldetag: **16.08.85**

(54) **Verfahren zur Herstellung von Pregnan-Derivaten.**

(30) Priorität: **17.09.84 DE 3434448**

(43) Veröffentlichungstag der Anmeldung:
**21.05.86 Patentblatt 86/21**

(45) Bekanntmachung des Hinweises auf die Patenterteilung:
**12.07.89 Patentblatt 89/28**

(84) Benannte Vertragsstaaten:
**AT BE CH DE FR GB IT LI LU NL SE**

(56) Entgegenhaltungen:
**EP-A-0 063 368**
**EP-A-0 123 241**
**DE-B-2 625 306**
**US-A-3 067 214**
**US-A-4 342 702**

**CHEMISCHE BERICHTE, Band 111, Nr. 9, September 1978, Seiten 3086-3093, Weinheim, DE; H. HOFMEISTER et al.: "Überführung von 17alpha-Ethinyl-17beta-nitrooxy-Steroiden in 17alpha-Hydroxy-20-oxopregnan-Derivate"**

(73) Patentinhaber: **SCHERING AKTIENGESELLSCHAFT**
**Berlin und Bergkamen, Müllerstrasse 170/178**
**Postfach 65 03 11, D-1000 Berlin 65 (DE)**

(72) Erfinder: **Hofmeister, Helmut, Dr., Weislingenstrasse 4, D-1000 Berlin 28 (DE)**
Erfinder: **Annen, Klaus, Dr., Osthofstrasse 80, D-4400 Münster- Albachten (DE)**
Erfinder: **Laurent, Henry, Dr., Glambecker Weg 21, D-1000 Berlin 28 (DE)**
Erfinder: **Wiechert, Rudolf, Prof., Petzower Strasse 8A, D-1000 Berlin 39 (DE)**

**EP 0 181 442 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft ein Verfahren zur Herstellung von Pregnan-Derivaten der allgemeinen Formel I

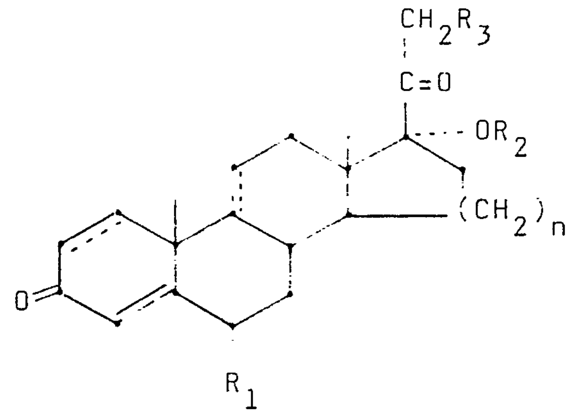

(I),

worin

..... jeweils eine Einfachbindung oder eine Doppelbindung symbolisiert,
n die Ziffern 1 oder 2 bedeutet,
$R_1$ ein Wasserstoffatom oder eine Methylgruppe darstellt,
$R_2$ ein Wasserstoffatom oder eine Formylgruppe bedeutet und
$R_3$ ein Chloratom, eine Hydroxygruppe oder eine Alkanoyloxygruppe mit bis zu 6 Kohlenstoffatomen darstellt, welches dadurch gekennzeichnet ist, daß man ein Androstan-Derivat der allgemeinen Formel II

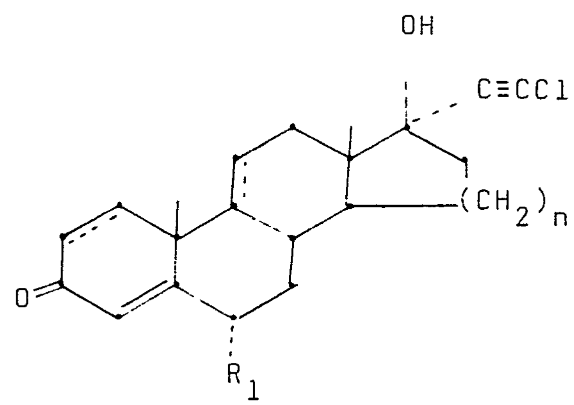

(II),

worin

....., n und $R_1$ die obengenannte Bedeutung besitzen, mittels Salpetersäure, Trifluoressigsäure oder Trichloressigsäure verestert, die Ester der allgemeinen Formel III

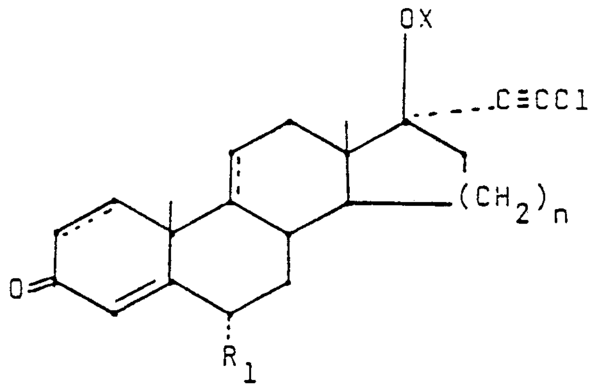

(III),

worin

....., n und $R_1$ die obengenannte Bedeutung besitzen und X eine Nitrogruppe, eine Trifluoracetylgruppe oder eine Trichloracetylgruppe darstellt, in Gegenwart von Silber(I)-Salzen mittels Ameisensäure zu Pregnan-Derivaten der allgemeinen Formel I a

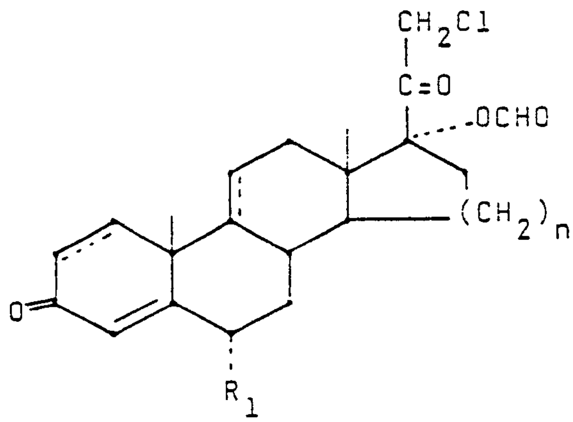

(Ia),

worin

....., n und $R_1$ die obengenannte Bedeutung besitzen, umsetzt und gewünschtenfalls die entstandenen 17-Formylester verseift und/oder das 21-Chloratom gegen eine Alkanoyloxygruppe austauscht und diese gegebenenfalls verseift.

Als Ausgangsmaterial für die Partialsynthese pharmakologisch wirksamer Steroide wird seit etwa zehn Jahren gewerbsmäßig zunehmend 4-Androsten-3,17-dion und 1,4-Androstadien-3,17-dion verwendet, die durch mikrobiologischen Seitenkettenabbau von Sterinen hergestellt werden. Dies hatte zur Folge, daß der Seitenkettenaufbau von Androstan-Derivaten zu Pregnan-Derivaten, der bereits vor dieser Zeit Gegenstand intensiver Forschungstätigkeit war (siehe beispielsweise J. Fried und J.A. Edwards: Organic Reactions in Steroid Chemistry, van Nostrand Reinhold Comp., New York, Volume II, 1976, 127-236) zunehmend an Bedeutung gewann. So gelang es beispielsweise 17α-Ethinyl-17β-nitrooxy-steroide (hergestellt durch Ethinylierung von 17-Oxosteroiden und Verestern der gebildeten 17α-Ethinyl-17β-hydroxysteroide mit Salpetersäure in Acetanhydrid) in 17β-Acetyl-17α-formyl-steroide zu überführen (Chem. Ber. 111, 1978, 3086-3096). Diese Verbindungen konnten mittels bekannter Verfahren in Pregnan-Derivate der allgemeinen Formel I gemäß Patentanspruch 1 überführt werden, deren weitere Umwandlung in pharmakologisch wirksame Steroide hinlänglich bekannt ist.

Mit Hilfe des erfindungsgemäßen Verfahrens ist es möglich, die Pregnan-Derivate der allgemeinen Formel I auf wesentlich einfacherem Wege zu synthetisieren, als dies mittels der vorbekannten Synthesen möglich war

Die erste Stufe des erfindungsgemäßen Verfahren kann unter den Bedingungen durchgeführt werden, die man üblicherweise zur Veresterung tertiärer oder sterisch gehinderter Steroidalkohole mit Saltptersäure, Trifluoressigsäure oder Trichloressigsäure anwendet. Die Veresterung mit Triflouressigsäure oder Trichloressigsäure kann beispielsweise unter völlig konventionellen Bedingungen mit den entsprechenden Trihalogenacetylchloriden oder Hexahalogenacetanhydriden in Gegenwart von Basen wie z. B. Pyridin durchgeführt werden. Zur Herstellung der Salpetersäureester hat sich als besonders geeignet die Veresterung mit Acetylnitrat (Tetrahedron 25, 1969, 761-769 modifiziert nach Chem. Ber. 111, 1978, 3086-3096) erwiesen. Das

hierzu benötigte Gemisch läßt sich beispielsweise aus den Komponenten darstellen (J. Amer. Chem. Soc., 82, 1960, 3588-3598). Geeignet ist Salpetersäure enthaltend etwa 70 bis 100 Gewichtsprozent Säure. Die Reaktion wird üblicherweise bei einer Reaktionstemperatur von -50°C bis ± 0°C, vorzugsweise -30°C bis 10°C durchgeführt. Die Reaktionszeit beträgt in der Regel 10 bis 120 Minuten.

Grundsätzlich kann der zweite Reaktionsschritt unter den in den obengenannten Publikationen beschriebenen Bedingungen oder Variationen derselben, wie sie im US-Patent 4 102 908 aufgeführt werden, durchgeführt werden. Für die gewerbsmäßige Durchführung sind diese Verfahrensbedingungen nicht sonderlich geeignet, da die notwendige Aufbereitung der Quecksilber(II)-Salze enthaltenen Abfälle recht aufwendig ist. Es ist wesentlich einfacher, diesen Reaktionsschritt unter Verwendung von Silber(I)-Salzen als Katalysator durchzuführen. Es ist in zweifacher Hinsicht überraschend, daß der Reaktionsschritt unter diesen Bedingungen in der gewünschten Weise abläuft. Aufgrund der Angaben in der Publikation in Chem Ber. hätte man einerseits erwarten sollen, daß bei der Verwendung von Silber(I)-Salzen, keine Hydratisierung der Ethinylgruppe eintreten würde. Andererseits war zu befürchten, daß das Chlorstom des Ausgangsprodukts oder Endprodukts abgespalten würde und den Silberkatalysator durch Bildung von Silberchlorid unwirksam macht.

Für diesen Verfahrensschritt werden als Katalysatoren vorzugweise gut dissoziierende Silber(I)-Salze wie beispeilsweise das Silber(I)-nitrat, das Silber(I)-acetat, das Silber/(I)-fluorid oder das Silber(I)-sulfat verwendet, die vozugsweise in einer Konzentration von 0,01 bis 0,5 mol % bezogen auf das Steroid eingesetzt werden. Dieser Reaktionsschritt wird vorzugsweise unter Verwendung konzentrierter Ameisensäure mit einem Gehalt von 95 bis 100 Gewichtsprozent Säure durchgefürt.

Ebenso wie bei der Umsetzung von Steroiden mit unsubstituierter 17α-Ethinyl-Seitenkette (Chem. Ber. III, 1978, 3086-3096) verläuft dieser Reaktionsschritt einheitlicher, wenn man der Reaktionsmischung zusätzlich ein dipolares aprotisches oder basisches Lösungsmittel zusetzt. Geeignete Zusätze sind beispielsweise tertiäre Amine, wie Triethylamin oder N-Methylmorpholin oder amidgruppenhaltige dipolare aprotische Lösungsmittel, wie Dimethylformamid, N-Methylacetamid oder auch insbesondere Hexamethylphosphorsäuretriamid oder 1-Methyl-2-pyrrolidon. Gute Ergebnisse erzielt man im allgemeinen, wenn man dem Reaktionsgemisch bezogen auf die übringen Komponenten 10 bis 50 % dieses Lösungsmittels zusetzt. Diese Reaktionsstufe wird üblicherweise bei einer Temperatur von 0°C bis 150°C durchgefürt.

Die sich gegebenenfalls anschließende Verseifung der 17-Formylester erfolgt unter den Bedingungen, wie sie dem Fachmann hinlänglich bekannt sind. Geeignet ist beispielweise die Verseifung dieser Verbindungen in gegebenenfalls einem wasserhaltigen niederen Alkohol (Methanol, Ethanol, Propanol oder Isopropanol) in Gegenwart basischer Katalysatoren (z. B. dem entsprechenden Natriumalkoholat oder Kaliumalkoholat, Natriumhydroxid, Kaliumhydroxid, Natriumkarbonat, Kaliumkarbonat oder Kaliumhydrogenkarbonat). Als Reaktionstemperatur wählt man in der Regel eine Temperatur zwischen 0°C und dem Siedepunkt des Lösungsmittels. Der sich gegebenenfalls anschließende Austausch des 21-Chloratoms gegen eine Alkanoyloxygruppe erfolgt ebenfalls unter konventionellen Bedingungen, so zum Beispiel in dem man die Verbindungen in einem inerten Lösungsmittel mit dem entsprechenden Natrium- oder Kalium-alkanoat umsetzt. Geeignete Lösungsmittel sind beispielsweise niedere Ketone, wie Aceton, Methylethylketon oder Methylisobutylketon oder dipolare aprotische Lösungsmittel wie, Dimethylformamid, N-Methylacetamid, Dimethylsulfoxid, 1-Methyl-2-pyrrolidon oder Hexamethylphosphorsäuretriamid. Die Reaktion wird in der Regel bei einer Temperatur von 50°C bis 120°C durchgeführt.

Die sich gegebenenfalls anschließende Verseifung der 21-Alkanoyloxyverbindungen kann unter den gleichen Bedingungen durchgeführt werden, wie die Verseifung der 17-Formylverbindungen.

Die Ausgangsverbindungen für das erfindungsgemäße Verfahren sind bekannt und können beispielsweise nach bekannten Methoden synthetisiert werden (Chem. Soc., 1962, 4995).

Die anschließenden Ausführungsbeispiele dienen zur Erläuterung des erfindungsgemäßen Verfahrens.

**Beispiel 1**

a) Zu einer Suspension von 5.7 g 17α-Chlorethinyl-17β-hydroxy-4-androsten-3-on in 50 ml Acetanhydrid tropft man bei -20°C 6,5 ml rauchende Salpetersäure. Nach 5 Minuten gibt man das Reaktionsgemisch in methanolhaltiges Eiswasser, saugt das ausgefallene Produkt ab, löst in Essigester, wäscht mit Wasser und trocknet über Natriumsulfat. Es werden 5.1 g 17α-Chlorethinyl-1782-nitrooxy-4-androsten-3-on erhalten. Schmelzpunkt 140°C (Zersetzung).

b) 4.0 g 17α-Chlorethinyl-17β-nitrooxy-4-androsten-3-on werden in 10 ml 1-Methyl-2-pyrrolidon gelöst. Bei 0°C gibt man 46 ml konz. Ameisensäure und 200 mg Silbernitrat zu und läßt bei Raumtemperatur rühren. Das Gemisch wird nach 8 Stunden in Eiswasser eingerührt. Das ausgefallene Produkt saugt man ab, löst in Essigester, wäscht mit Wasser und trocknet über Natriumsulfat. Es werden 3.2 g 21-Chlor-17-formyloxy-4-pregnen-3,20-dion erhalten. Schmelzpunkt 204,9°C.

**Beispiel 2**

1.6 g 21-Chlor-17-formyloxy-4-pregnen-3,20-dion werden bei Raumtemperatur in einem Gemisch aus 60 ml Methanol und 9 ml Wasser mit 500 mg Kaliumhydrogencarbonat gerührt. Nach 1 Stunde wird das Reaktionsgemisch in Eiswasser eingerührt. Das ausgefallene Produkt wird abgesaugt, mit Wasser gewaschen und über Natriumsulfat getrocknet. Man erhält 1.3 g 21-Chlor-17-hydroxy-4-pregnen-3,20-dion. Schmelzpunkt 239,4° C.

**Beispiel 3**

1,0 g 21-Chlor-17-hydroxy-4-pregnen-3,20-dion in 20 ml Dimethylformamid werden mit 1.0 g Kaliumacetat bei 80° C gerührt. Nach 30 Minuten gibt man das Reaktionsgemisch in Eiswasser, saugt das ausgefallene Produkt ab, löst in Essigester und trocknet über Natriumsulfat. Es werden 940 mg 21-Acetoxy-17-hydroxy-4-pregnen-3,20-dion erhalten. Schmelzpunkt 236,5° C.

**Beispiel 4**

a) 11.5 g 4,9(11)-Androstadien-3,17-dion [US-Pat. 3 441 559 (1969)] in 200 ml Dioxan werden mit 20 ml Orthoameisensäuretrimethylether und 100 mg p-Toluolsulfonsäure umgesetzt. Nach 48 Stunden gibt man zur Lösung 5 ml Pyridin, engt im Vakuum ein, nimmt den Rückstand in Essigester auf, wäscht mit Wasser und trocknet über Natriumsulfat. Nach Chromatographieren des Rohproduktes mit einem Hexan-Essigester-Gradienten an Kieselgel, das 2 % Triethylamin enthält, isoliert man 9.2 g 3-Methoxy-3,5,9(11)-androstatrien-17-on. Schmelzpunkt 153,4° C.

b) Zu 6 ml 1,2-Dichlorethylen in 100 ml abs. Ether werden bei 0° C 80 ml einer 1.5 mol. Methyllithium-Lösung (in Ether) getropft. Nach 30 Min. gibt man langsam 6.3 g 3-Methoxy-3,5,9(11)-androstatrien 17-on in 150 ml abs. Tetrahydrofuran zu. Das Reaktionsgemisch wird nach 15 min mit Ether verdünnt und vorsichtig mit 50 ml gesättigter Ammoniumchloridlösung versetzt. Die organische Phase wird mit 2 N Salzsäure und Wasser neutral gewaschen, über Natriumsulfat getrocknet und im Vakuum eingeengt. Das erhaltene Rohprodukt wird in 30 ml Aceton bei Raumtemperatur mit 0.3 ml 70 %-ige Perchlorsäure versetzt. Nach 30 Min. wird das Reaktionsgemisch in Eiswasser gegeben, das ausgefallene Produkt wird abgesaugt, in Essigester gelöst, mit Wasser gewaschen und über Natriumsulfat getrocknet. Nach Chromatographieren des Rohproduktes an Kieselgel mit einem Hexan-Essigester-Gradienten werden 6.0 g 17α-Chlorethinyl-17β-hydroxy-4,9(11)-androstadien-3-on erhalten. Schmelzpunkt 157,1° C.

c) 5.7 g 17α-Chlorethinyl-17β-hydroxy-4,9(11)-androstadien-3-on werden analog Beispiel 1a mit rauchender Salpetersäure in Acetanhydrid umgesetzt. Nach Chromatographieren des Rohproduktes an Kieselgel mit einem Hexan-Essigester-Gradienten erhält man 4.8 g 17α-Chlorethinyl-17β-nitrooxy-4,9(11)-androstadien-3-on als Schaum.

d) 4.5 g 17α-Chlorethinyl-17β-nitrooxy-4,9(11)-androstadien-3-on werden analog Beispiel 1b umgesetzt. Es werden 3.8 g 21-Chlor-17-formyloxy-4,9(11)-pregnadien-3,20-dion erhalten. Schmelzpunkt 202,7° C.

**Beispiel 4**

2.5 g 21-Chlor-17-formyloxy-4,9(11)-pregnadien-3,20-dion werden analog Beispiel 2 umgesetzt. Man erhält 2.2 g 21-Chlor-17-hydroxy-4,9(11)-pregnadien-3,20-dion. Schmelzpunkt 236,9° C.

**Beispiel 5**

1.5 g 21-Chlor-17-hydroxy-4,9(11)-pregnadien-3,20-dion werden analog Beispiel 3 umgesetzt. Es werden 1.4 g 21-Acetoxy-17-hydroxy-4,9(11)-pregnadien-3,20-dion erhalten. Schmelzpunkt 234,3° C.

**Beispiel 6**

a) 1.5 g 21-Acetoxy-17-hydroxy-4,9(11)-pregnadien-3,20-dion in 40 ml Methanol werden bei Raumtemperatur mit 15 ml 0.2 N methanolischer Kaliumhydroxidlösung 2 h bei Raumtemperatur gerührt. Das Reaktionsgemisch wird in Eiswasser gegeben. Man saugt das ausgefallene Produkt ab, löst in Methylenchlorid, wäscht mit

Wasser und trocknet über Natriumsulfat. Es werden 1.2 g 17,21-Dihydroxy-4,9(11)-pregnadien-3,20-dion vom Schmelzpunkt 248,2° C erhalten.

b) 20.0 g 17α-Chlorethinyl-17β-hydroxy-4-androsten-3-on in 1 l Dioxan werden mit 20.0 g 2,3-Dichlor-5,6-dicyan-p-benzochinon bei 110° C gerührt. Nach 20 Stunden wird mit Methylenchlorid verdünnt, mit Wasser und Natriumhydrogencarbonatlösung gewaschen und über Natriumsulfat getrocknet. Das Rohprodukt wird an Kieselgel mit einem Hexan-Essigester-Gradienten chromatographiert. Ausbeute 14.7 g 17α-Chlorethinyl-17β-hydroxy-1,4-androstadien-3-on. Schmelzpunkt 125,2° C.

c) 8.0 g 17α-Chorethinyl-17β-hydroxy-1,4-androstadien-3-on werden analog Beispiel 1a mit rauchender Salpetersäure umgesetzt. Es werden 6.8 g 17α-Chlorethinyl-17β-nitrooxy-1,4-androstadien-3-on isoliert. Schaum.

d) 2.6 g 17α-Chlorethinyl-17β-nitrooxy-1,4-androstadien-3-on werden analog Beispiel 1a umgesetzt. Es werden 1.8 g 21-Chlor-17-formyloxy-1,4-pregnadien-3,20-dion erhalten.

**Beispiel 7**

1.2 g 21-Chlor-17-formyloxy-1,4-pregnadien-3,20-dion werden analog Beispiel 2 umgesetzt. Es werden 960 mg 21-Chlor-17-hydroxy-1,4-pregnadien-3,20-dion erhalten.

**Beispiel 8**

820 mg 21-Chlor-17-hydroxy-1,4-pregnadien-3,20-dion werden analog Beispiel 3 zu 21-Acetoxy-17-hydroxy-1,4-pregnadien-3,20-dion umgesetzt. Ausbeute 710 mg, Schmelzpunkt 215,8° C.

**Beispiel 9**

a) 16.0 g 17α-Chlorethinyl-17β-hydroxy-4,9(11)-pregnadien-3-on werden analog Beispeil 6b mit 2,3-Dichlor-5,6-dicyan-p-benzochinon in Dioxan umgesetzt. Man erhält 9.8 g 17α-Chlorethinyl -17β-hydroxy-1,4,9(11)-androstatrien-3-on.

b) 8.6 g 17α-Chlorethinyl-17β-hydroxy-1,4,9(11)-androstatrien-3-on werden analog Beispiel 1a mit rauchender Salpetersäure umgesetzt. Man erhält 7.8 g 17α-Chlorethinyl-17β-nitrooxy-1,4,9(11)-androstatrien-3-on.

c) 6.5 g 17α-Chlorethinyl-17β-nitrooxy-1,4,9(11)-androstatrien-3-on werden analog Beispiel 1b umgesetzt. Man erhält 4.9 g 21-Chlor-17-formyloxy-1,4,9(11)-pregnatrien-3,20-dion.

**Beispiel 10**

3.2 g 21-Chlor-17-formyloxy-1,4,9(11)-pregnatrien-3,20-dion werden analog Beispiel 2 zu 21-Chlor-17-hydroxy-1,4,9(11)-pregnatrien-3,20-dion umgesetzt. Ausbeute 2.9 g, Schmelzpunkt 190,5° C.

**Beispiel 11**

1.2 g 21-Chlor-17-hydroxy-1,4,9(11)-pregnatrien-3,20-dion werden analog Beispiel 3 umgesetzt. Es werden 980 mg 21-Acetoxy-17-hydroxy-1,4,9(11)-pregnatrien-3,20-dion erhalten.

**Beispiel 12**

a) Analog Beispiel 4a werden 7.5 g 6α-Methyl-4,9(11)-androstadien-3,17-dion zu 3-Methoxy-6-methyl-3,5,9(11)-androstatrien-17-on umgesetzt. Ausbeute 6.1 g.

b) Aus 5.5 g 3-Methoxy-6-methyl-3,5,9(11)-androstatrien-17-on werden analog Beispiel 4b 5.1 g 17α-Chlorethinyl-17β-hydroxy-6α-methyl-4,9(11)-androstadien-3-on erhalten.

c) 4.5 g 17α-Chlorethinyl-17β-hydroxy-6α-methyl-4,9(11)-androstadien 3-on werden analog Beispiel 1a zu 17α-Chlorethinyl-17β-nitrooxy-6α-methyl-4,9(11)-androstadien-3-on umgesetzt. Ausbeute 3.8 g.

d) 2.9 g 17α-Chlorethlnyl-17β-nitrooxy-6α-methyl-4,9(11)-androstadien-3-on werden analog Beispiel 1b zu 21-Chlor-17-formyloxy-6α-methyl-4,9(11)-pregnadien-3,20-dion umgesetzt. Ausbeute 2.1 g.

**Beispiel 13**

Analog Beispiel 2 werden 1.6 g 21-Chlor-17-formyloxy-6α-methyl-4,9(11)-pregnadien-3,20-dion zu 21-Chlor-17-hydroxy-6α-methyl-4,9(11)-pregnadien-3,20-dion umgesetzt. Ausbeute 1.3 g.

**Beispiel 14**

a) Aus 1.1 g 21-Chlor-17-hydroxy-6α-methyl-4,9(11)-pregnadien-3,20-dion erhält man nach Beispiel 3 870 mg 21-Acetoxy-17-hydroxy-6α-methyl-4,9(11)-pregnadien-3,20-dion.

**Beispiel 15**

a) 12.5 g 3β-Hydroxy-D-homo-5-androsten-17a-on werden analog Beispiel 4b mit Lithiumchloracetylid umgesetzt. Nach Chromatographieren des Rohproduktes an Kieselgel mit einem Hexan-Aceton-Gradienten werden 8.9 g 17aα-Chlorethinyl-D-homo-5-androsten-3β, 17aβ-diol erhalten.

b) Aus einer Lösung von 10.0 g 17aα-Chlorethinyl-D-homo-5-androsten-3β, 17aβ-diol in 300 ml Toluol und 50 ml Cyclohexanon werden etwa 5 ml Lösungsmittel abdestilliert. Unter weiterem Abdestillieren von Lösungsmittel werden 4.0 g Aluminiumtriisopropylat in 30 ml Toluol zugetropft. Nach 1.5 h gibt man langsam 50.0 g Kaliumnatriumtartrat in 70 ml Wasser zu und rührt 30 min unter Rückfluß. Nach dem Abkühlen der Lösung verdünnt man mit Essigester, wäscht die organische Phase mehrmals mit Wasser und trocknet über Natriumsulfat. Nach Chromatographieren des Rohproduktes an Kieselgel mit einem Hexan-Aceton-Gradienten erhält man 8.4 g 17aα-Chlorethinyl -17aβ-hydroxy-D-homo-4-androsten-3-on.

c) 8.3 g 17aα-Chlorethinyl-17aβ-hydroxy-D-homo-4-androsten-3-on werden analog Beispiel 1a zu 17aα-Chlorethinyl-17aβ-nitrooxy-D-homo-4-androsten-3-on umgesetzt. Ausbeute 6.7 g.

d) 6,3 g 17aα-Chlorethinyl-17aβ-nitrooxy-D-homo-4-androsten-3-on werden analog Beispiel 1b umgesetzt. Es werden 4.3 g 21-Chlor-17aα-formyloxy-D-homo-4-pregnen-3,20-dion isoliert.

**Beispiel 16**

3.8 g 21-Chlor-17aα-formyloxy-D-homo-4-pregnen-3,20-dion werden analog Beispiel 2 zu 21-Chlor-17aα-hydroxy-D-homo-4-pregnen-3,20-dion umgesetzt. Ausbeute 3.1 g.

**Beispiel 17**

2.4 g 21-Chlor-17aα-hydroxy-D-homo-4-pregnen-3,20-dion werden analog Beispiel 3 zu 21-Acetoxy-17aα-hydroxy-D-homo-4-pregnen-3,20-dion umgesetzt. Ausbeute 2.1 g.

**Beispiel 18**

a) Zu 5.0 g 17α-Chlorethinyl-17β-hydroxy-4-androsten-3-on in 25 ml Pyridin tropft man bei 0°C 3.4 ml Triflouressigsäureanhydrid. Nach 30 min gibt das Reaktionsgemisch in salzsaures Eis/Wasser. Das ausgefallene Produkt wird abgesaugt, mit Wasser gewaschen und getrocknet. Nach Chromatographieren des Rohproduktes an Kieselgel mit Hexan/Essigester werden 4.1 g 17α-Chloethinyl-17β-triflour-acetoxy-4-androsten-3-on erhalten. Schmelzpunkt: 139°C.

b) 3.5 g 17α-Chlorethinyl-17β-triflouracetoxy-4-androsten-3-on werden in 30 ml konz. Ameisensäure und 6 ml 1-Methyl-2-pyrrolidon mit 300 mg Silbernitrat bei 60°C gerürt. Nach 6 stunden gibt man das Reaktionsgemisch in Eiswasser. Das ausgefallene Produkt wird abgesaugt, mit Wasser gewaschen und getrocknet. Nach Chromatographieren des Rohproduktes an Kieselgel mit Hexan/Essigester erhält man 1.3 g 21-Chlor-17-formyloxy-4-pregnen-3,20-dion. Schmelzpunkt: 201.5°C.

**Beispiel 19**

a) Zu 5.0 g 17α-Chlorethinyl-17β-hydroxy-4-androsten-3-on in 30 ml Pyridin werden 3.5 ml Trichloressigsäureanhydrid bei 0°C getropft. Nach 15 min gibt man das Reaktionsgemisch in schwefelsäurehaltiges Eis/Wasser. Das ausgefallene Produkt wird in Essigester aufgenommen. Nach Chromatographieren des Rohproduktes an Kiselgel mit Hexan/Essigester erhält man 4.8 g 17α-Chlorethinyl-17β-trichloracetoxy-4-androsten-3-on. Schmelzpunkt: 173°C (Zers.)

b) 1.4 g 17α-Chlorethinyl-17β-trichloracetoxy-4-androsten-3-on werden - wie im Beispiel 18a) beschrieben - in konz. Ameisensäure und 1-Methyl-2-pyrrolidon mit Silbernitrat umgesetzt. Nach Chromatographieren des Rohproduktes an Kiesegel mit Hexan/Essigester werden 630 mg 21-Chlor-17-formyloxy-4-pregnen-3,20-dion erhalten. Schmelzpunkt: 202.5°C.

**Patentansprüche**

1. Verfahren zur Herstellung von Pregnan-Derivaten der allgemeinen Formel I

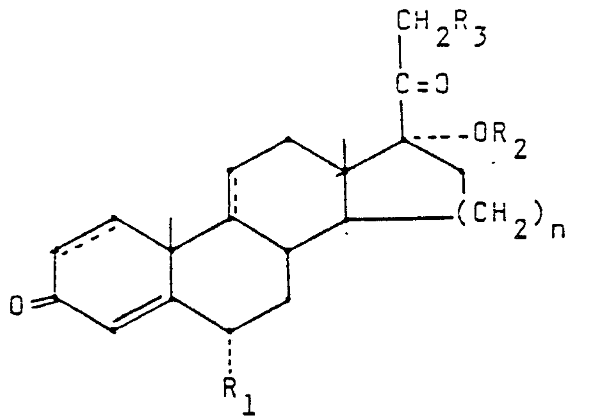

(I),

worin

..... jeweils eine Einfachbindung oder eine Doppelbindung symbolisiert,
n die Ziffern 1 oder 2 bedeutet,
$R_1$ ein Wasserstoffatom oder eine Methylgruppe darstellt,
$R_2$ ein Wasserstoffatom oder eine Formylgruppe bedeutet und
$R_3$ ein Chloratom, eine Hydroxygruppe oder eine Alkanoyloxygruppe mit bis zu 6 Kohlenstoffatomen darstellt, dadurch gekennzeichnet, daß man ein Androstan-Derivat der allgemeinen Formel II

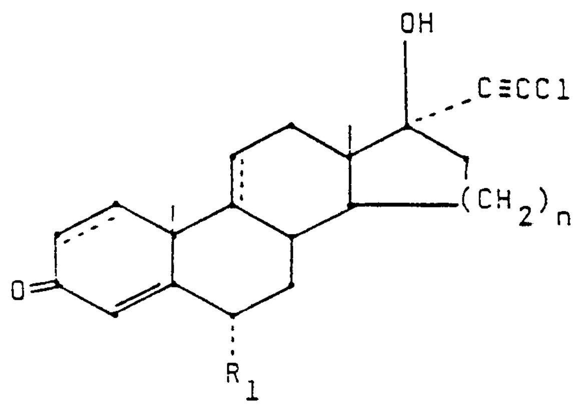

(II),

worin

....., n und $R_1$ die obengenannte Bedeutung besitzen, mittels Salpetersäure, Trifluoressigsäure oder Trichloressigsäure verestert, die Ester der allgemeinen Formel III

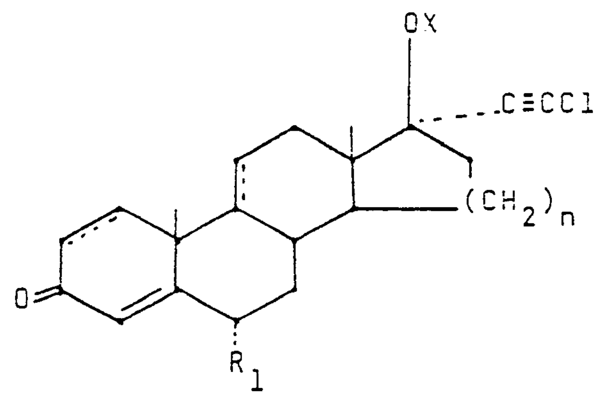

(III),

worin

....., n und $R_1$ die obengenannte Bedeutung besitzen und X eine Nitrogruppe, eine Trifluoracetylgruppe oder eine Trichloracetylgruppe darstellt, in Gegenwart von Silber(I)-Salzen mittels Ameisensäure zu Pregnan-Derivaten der allgemeinen Formel Ia

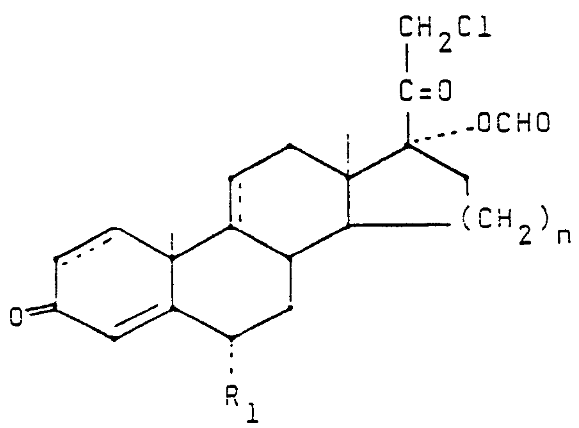

(Ia),

worin

....., n und $R_1$ die obengenannte Bedeutung besitzen, umsetzt und gewünschtenfalls die entstandenen 17-Formylester verseift und/oder das 21-Chloratom gegen eine Alkanoyloxygruppe austauscht und diese gegebenenfalls verseift.

2. Vefahren zur Herstellung von Pregnan-Derivaten der allgemeinen Formel I gemäß Patentanspruch 1, dadurch gekennzeichnet, daß man zur Veresterung der Androstan-Derivate der allgemeinen Formel II mit Salpetersäure Acetylnitrat verwendet.

3. Verfahren zur Herstellung von Pregnan-Derivaten der allgemeinen Formel I gemäß Anspruch 1 und 2, dadurch gekennzeichnet, daß man zur Umsetzung der Ester der allgemeinen Formel III mit Ameisensäure 0,01 bis 0,5 mol eines dissoziierenden Silber(I)-Salzes bezogen auf das Steroid als Katalysator verwendet.

4. Verfahren zur Herstellung von Pregnan-Derivaten der allgemeinen Formel I gemäß Patentanspruch 1 bis 3, dadurch gekennzeichnet, daß man die Umsetzung der Ester der allgemeinen Formel III in einem Gemisch aus Ameisensäure und einem dipolaren aprotischen oder basischen Lösungsmittel durchführt.

5. Ester der allgemeinen Formel III

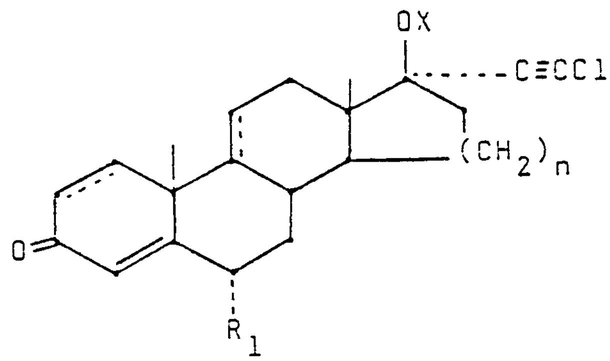

(III),

worin

..... jeweils eine Einfachbindung oder eine Doppelbindung symbolisiert,
n die Ziffer 1 oder 2 bedeutet
$R_1$ ein Wasserstoffatom oder eine Methylgruppe darstellt, und X eine Nitrogruppe, eine Trifluoracetylgruppe oder eine Trichloracetylgruppe bedeutet.

6. 17α-Chlorethinyl-17β-nitrooxy-4-androsten-3-on.
7. 17α-Chlorethinyl-17β-nitrooxy-4,9(11)-androstadien-3-on.
8. 17α-Chlorethinyl-17β-nitrooxy-1,4-androstadien-3-on.
9. 17α-Chlorethinyl-17β-nitrooxy-1,4,9(11)-androstatrien-3-on.
10. 17α-Chlorethinyl-17β-nitrooxy-6α-methyl-4,9(11)-androstadien-3-on.
11. 17aα-Chlorethinyl-l7aβ-nitrooxy-D-homo-4-androsten-3-on.
12. 17α-Chlorethinyl-17β-trifluoracetyloxy-4-androsten-3-on.
13. 17α-Chlorethinyl-17β-trichloracetyloxy-4-androsten-3-on.

**Claims**

1. Process for the preparation of pregnane derivatives of the general formula I

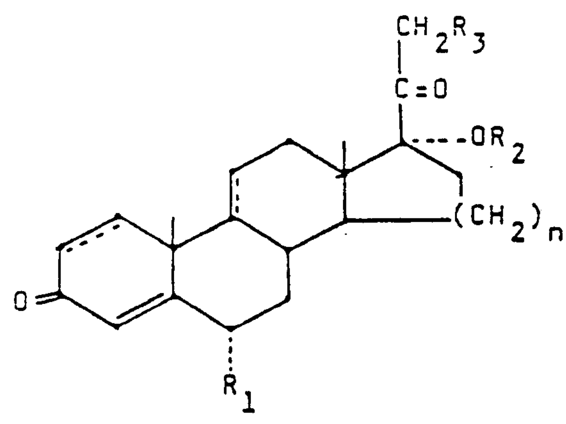

(I)

wherein
..... in each case symbolises a single bond or a double bond,
n represents the number 1 or 2,
$R_1$ represents a hydrogen atom or a methyl group,
$R_2$ represents a hydrogen atom or a formyl group and
$R_3$ represents a chlorine atom, a hydroxy group or an alkanoyloxy group having up to 6 carbon atoms, characterised in that an androstane derivative of the general formula II

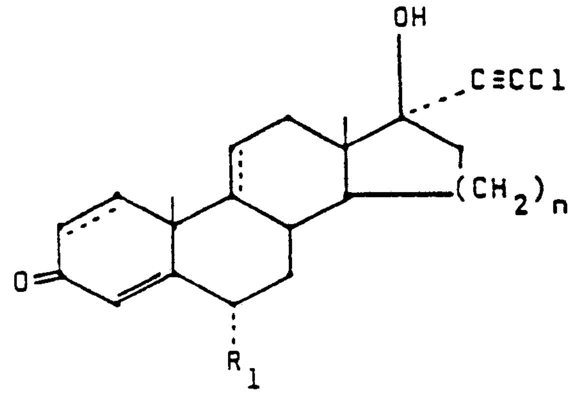

(II)

wherein

....., $\underline{n}$ and $R_1$ have the meanings mentioned above, is esterified by means of nitric acid, trifluoroacetic acid or trichloroacetic acid, the esters of the general formula III

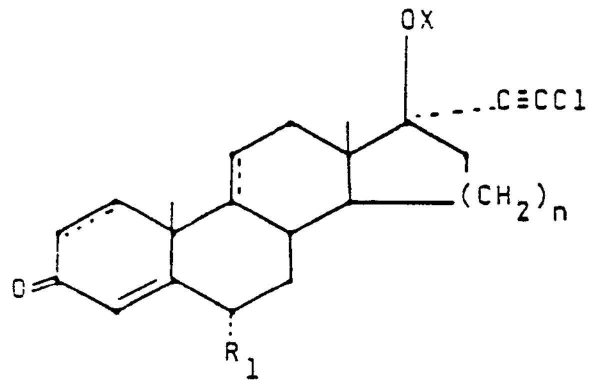

(III)

wherein

....., n and $R_1$ have the meanings mentioned above and X represents a nitro group, a trifluoroacetyl group or a trichloroacetyl group, are reacted in the presence of silver (I) salts by means of formic acid to form pregnane derivatives of the general formula Ia

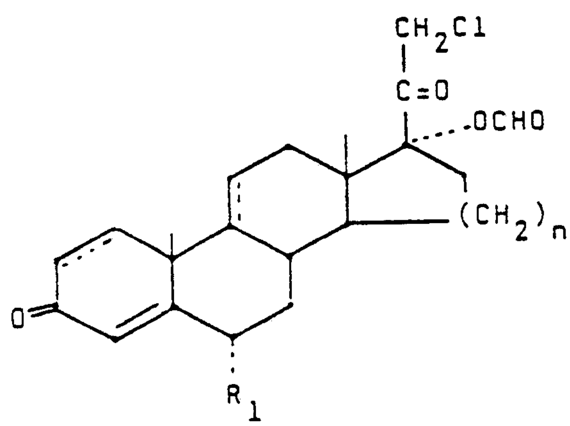

(Ia)

wherein

....., $\underline{n}$ and $R_1$ have the meanings mentioned above, and, if desired, the resulting 17-formyl esters are hydrolysed and/or the 21-chlorine atom is exchanged for an alkanoyloxy group and the latter is optionally hydrolysed.

2. Process for the preparation of pregnane derivatives of the general formula I according to patent claim 1,

characterised in that acetyl nitrate is used for the esterification of the androstane derivatives of the general formula II with nitric acid.

3. Process for the preparation of pregnane derivatives of the general formula I according to claims 1 and 2, characterised in that from 0.01 to 0.5 mol of a dissociating silver(I) salt, based on the steroid, is used as catalyst for the reaction of the esters of the general formula III with formic acid.

4. Process for the preparation of pregnane derivatives of the general formula I according to patent claims 1 to 3, characterised in that the reaction of the esters of the general formula III is carried out in a mixture of formic acid and a dipolar aprotic or basic solvent.

5. Esters of the general formula III

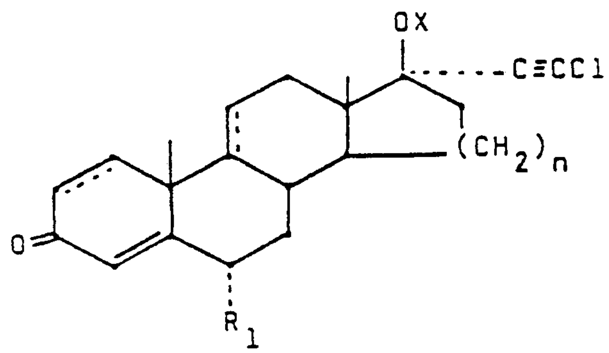

(III)

wherein

..... in each case symbolises a single bond or a double bond,
n represents the number 1 or 2,
$R_1$ represents a hydrogen atom or a methyl group, and
X represents a nitro group, a trifluoroacetyl group or a trichloroacetyl group.

6. 17α-chloroethynyl-17β-nitrooxy-4-androsten-3-one.
7. 17α-chloroethynyl-17β-nitrooxy-4,9(11)-androstadien-3-one.
8. 17α-chloroethynyl-17β-nitrooxy-1,4-androstadien-3-one.
9. 17α-chloroethynyl-17β-nitrooxy-1,4,9(11)-androstatrien-3-one.
10. 17α-chloroethynyl-17β-nitrooxy-6α-methyl-4,9(11)-androstadien-3-one.
11. 17aα-chloroethynyl-17aβ-nitrooxy-D-homo-4-androsten-3-one.
12. 17α-chloroethynyl-17β-trifluoroacetoxy-4-androsten-3-one.
13. 17α-chloroethynyl-17β-trichloroacetoxy-4-androsten-3-one.

## Revendications

1. Procédé pour préparer des dérivés du prégnane répondant à la formule générale I:

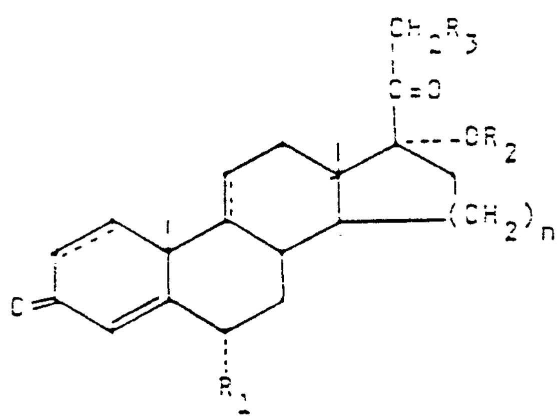

(I)

dans laquelle

les symboles ...... représentent chacun une liaison simple ou une liaison double,

n représente le nombre 1 ou le nombre 2,
$R_1$ représente un atome d'hydrogène ou un radical méthyle,
$R_2$ représente un atome d'hydrogène ou un radical formyle et
$R_3$ représente un atome de chlore, un radical hydroxy ou un radical alcanoyloxy contenant au maximum 6 atomes de carbone,
procédé caractérisé en ce qu'on estérifie un dérivé de l'androstane répondant à la formule générale II:

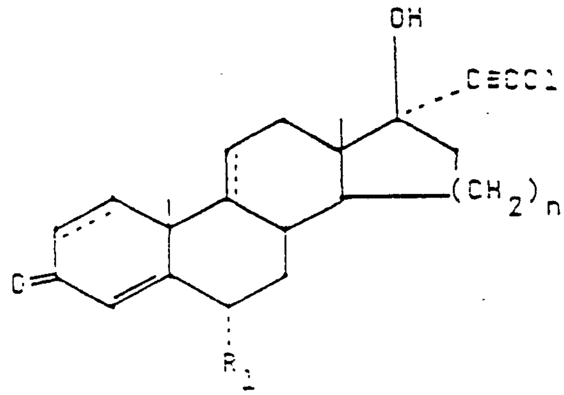
(II)

dans laquelle ......., n et $R_1$ ont les significations qui leur ont été données ci-dessus,
au moyen d'acide nitrique, d'acide trifluoracétique ou d'acide trichloracétique, on transforme les esters répondant à la formule générale III :

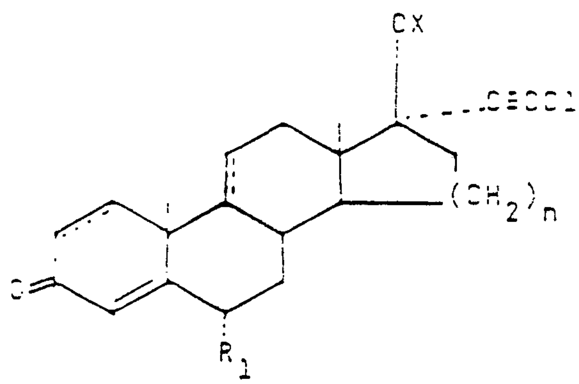
(III)

dans laquelle ......, n et $R_1$ ont les significations qui leur ont été données ci-dessus, et X représente un radical nitro, trifluoracétyle ou trichloracétyle,
en présence de sels d'argent (I), au moyen d'acide formique, en dérivés du prégnane répondant à la formule générale Ia:

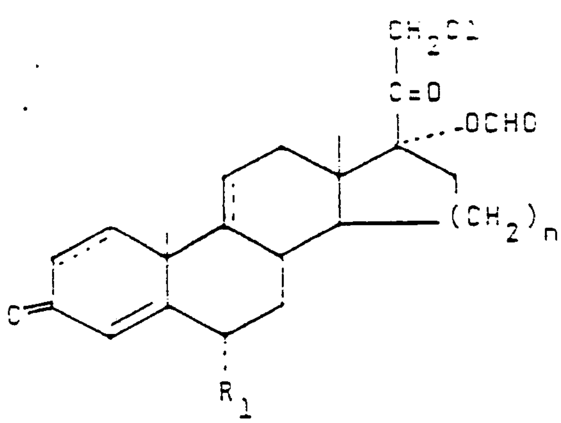
(Ia)

dans laquelle ....., n et $R_1$ ont les significations qui leur ont été données ci-dessus,

et, si on le désire, on saponifie les esters formyliques en 17 formés et/ou on échange l'atome de chlore en 21 contre un radical alcanoyloxy et on saponifie éventuellement ce dernier.

2. Procédé de préparation de dérivés du prégnane de formule générale I selon la revendication 1, procédé caractérisé en ce que, pour estérifier les dérivés de l'androstane de formule générale II avec l'acide nitrique, on utilise le nitrate d'acétyle.

3. Procédé de préparation de dérivés du prégnane de formule générale I selon l'une des revendications 1 et 2, procédé caractérisé en ce que, pour faire réagir l'ester de formule générale III avec l'acide formique, on utilise, comme catalyseur, de 0,01 à 0,5 mol d'un sel d'argent(I) apte à se dissocier, par rapport au stéroïde.

4. Procédé de préparation de dérivés du prégnane de formule générale I selon l'une quelconque des revendications 1 à 3, procédé caractérisé en ce qu'on effectue la réaction des esters de formule générale III dans un mélange d'acide formique et d'un solvant dipolaire aprotique ou basique.

5. Esters répondant à la formule générale III:

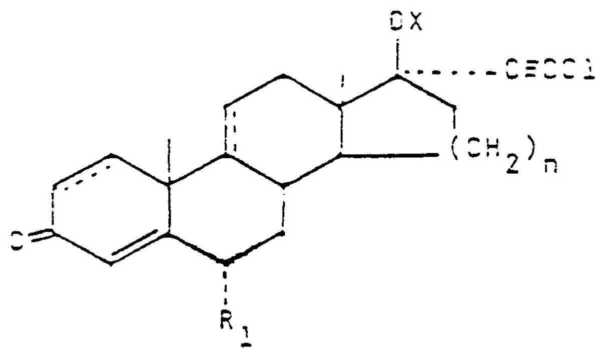

(III)

dans laquelle
les symboles ...... représentent chacun une liaison simple ou une liaison double,
n est égal à 1 ou à 2,
$R_1$ représente un atome d'hydrogène ou un radical méthyle, et
X représente un radical nitro, trifluoracétyle ou trichloracétyle.

6. Chloréthynyl-17α nitro-oxy-17β androstène-4 one-3.
7. Chloréthynyl-17α nitro-oxy-17β androstadiène-4,9(11) one-3.
8. Chloréthynyl-17α nitro-oxy-17β androstadiène-1,4 one-3.
9. Chloréthynyl-17α nitro-oxy-17β androstatriène-1,4,9(11) one-3.
10. Chloréthynyl-17α nitro-oxy-17β méthyl-6α androstadiène-4,9(11) one-3.
11. Chloréthynyl-17aα nitro-oxy-17aβ D-homoandrostène-4 one-3.
12. Chloréthynyl-17α trifluoracétyloxy-17β androstène-4 one-3.
13. Chloréthynyl-17α trichloracétyloxy-17β androstène-4 one-3.